# EUROPÄISCHE PATENTSCHRIFT

(11) **EP 3 699 342 B1**
(45) Veröffentlichungstag und Bekanntmachung des Hinweises auf die Patenterteilung: **17.11.2021**
(21) Anmeldenummer: 19158771.6
(22) Anmeldetag: 22.02.2019
(51) Int. Cl.: D04B 1/26, D04B 1/18, D04B 1/10, A61F 13/06, A61F 13/08, A61F 13/10

(54) **VERFAHREN ZUR HERSTELLUNG EINES GESTRICKTEILS SOWIE GESTRICKTEIL**
METHOD FOR MANUFACTURING A KNITTED PART AND KNITTED PART
PROCÉDÉ DE FABRICATION D'UNE PIÈCE TRICOTÉE AINSI QUE PIÈCE TRICOTÉE

(43) Veröffentlichungstag der Anmeldung: 26.08.2020
(73) Patentinhaber: medi GmbH & Co. KG, 95448 Bayreuth (DE)
(72) Erfinder: KÜBRICH, Wolfgang, 96215 Lichtenfels (DE)
(74) Vertreter: Lindner Blaumeier Patent- und Rechtsanwälte

(56) Entgegenhaltungen:
- WO-A1-97/39172
- DE-A1- 4 237 389
- US-A1- 2007 051 137

## Beschreibung

Die Erfindung betrifft ein Verfahren zur Herstellung eines Gestrickteils aus wenigstens einem Maschen bildenden Gestrickfaden und wenigstens einem elastischen Schussfaden auf einer ein erstes und ein zweites Nadelbett aufweisenden Strickmaschine.

Gestrickteile, beispielsweise in Form eines Bein- oder Armstrumpfes oder einer Bandage etc., werden häufig über ein Gelenk gezogen. Beim Abwinkeln des Gelenks kommt es in der Gelenkbeuge zu einem Zusammenschieben des Gestrickmaterials und zu einer Faltenbildung. Diese Faltenbildung, beispielsweise im Bereich der Kniekehle oder der Armbeuge, wird häufig als unangenehm empfunden, insbesondere wenn über das Gestrickteil auch ein gewisser Druck auf das darunter liegende Gewebe ausgeübt werden soll, wie beispielsweise bei Ausführung des Gestrickteils als kompressives Gestrickteil, z. B. in Form eines Kompressionsstrumpfes, wobei ein kompressives Gestrickteil einen definierten Druck auf das darunter befindliche Gewebe ausüben muss, wie beispielsweise in den Güte- und Prüfbestimmungen für medizinische Kompressionsstrümpfe gemäß der Gütesicherung RAL-GZ 38771 "Medizinische Kompressionsstrümpfe" definiert ist. Ein solches kompressives Gestrickteil unterscheidet sich also von einem üblichen, etwas elastischeren Gestrickteil.

Zur Vermeidung der Faltenbildung offenbart die WO 97/39172 ein Gestrick mit einer Rippenstruktur auf beiden Warenseiten sowie die DE 42 37 389 ein Gestrick mit einer Wellenstruktur auf einer Warenseite. Die US 2007/0051137 zeigt ein Schlauchgestrick mit durch den Einsatz der Spickeltechnik hergestellten, dehnbaren Wellen mit Stützwirkung.

Der Erfindung liegt damit das Problem zugrunde, ein Verfahren zur Herstellung eines demgegenüber verbesserten Gestrickteils anzugeben.

Zur Lösung dieses Problems ist erfindungsgemäß ein Verfahren zur Herstellung eines Gestrickteils aus wenigstens einem Maschen bildenden Gestrickfaden und wenigstens einem elastischen Schussfaden auf einer ein erstes und ein zweites Nadelbett aufweisenden Strickmaschine vorgesehen, mit folgenden Schritten:
a) Stricken wenigstens einer Maschenreihe mit rechts-rechts-Bindung auf beiden Nadelbetten mit dem Gestrickfaden,
b) Einlegen des elastischen Schussfadens,
c) Abstricken der Maschen auf dem ersten Nadelbett mit dem Gestrickfaden mit einer rechts-links-Bindung,
d) Umhängen der auf dem ersten Nadelbett befindlichen Maschen auf das zweite Nadelbett,
e) Abstricken der Maschen auf dem zweiten Nadelbett mit dem Gestrickfaden mit einer rechts-links-Bindung,
f) Stricken wenigstens einer Maschenreihe mit rechts-rechts-Bindung auf beiden Nadelbetten mit dem Gestrickfaden,
g) Einlegen des elastischen Schussfadens,
h) Abstricken der Maschen auf dem zweiten Nadelbett mit dem Gestrickfaden mit einer rechts-links-Bindung,
i) Umhängen der auf dem zweiten Nadelbett befindlichen Maschen auf das erste Nadelbett,
j) Abstricken der Maschen auf dem ersten Nadelbett mit dem Gestrickfaden mit einer rechts-links-Bindung,
k) beliebig häufiges Wiederholen der Schritte a) ― j).

Dieses erfindungsgemäße Herstellungsverfahren eines Gestrickteils ermöglicht die Ausbildung eines Strukturabschnitts bestehend aus mehreren parallel zueinander liegenden, gesehen in Gestricklängsrichtung, also in Richtung der Maschenstäbchen mäanderförmig verlaufenden Maschenreihen, die bei Einwirken einer in Längsrichtung des Gestricks wirkende Zugbeanspruchung unter Aufbau einer Rückstellkraft auseinandergezogen werden können, und die bei Einwirken einer entgegengesetzt wirkenden Druckspannung gegeneinander verschiebbar sind, ohne dabei in größerem Umfang Falten zu bilden. Es ergibt sich also eine Reihen- oder Rippenstruktur, wobei das Gestrickteil von beiden Seiten identisch ausschaut, lediglich sind die einzelnen Rippen in Richtung der Maschenstäbchen versetzt zueinander und befinden sich in verschiedenen Ebenen. Dies ermöglicht es, dass bei Abwinkeln des Gelenks in der Gelenkbeuge, in welchem Bereich zumindest dieser Strukturabschnitt ausgebildet ist, die einzelnen Maschenreihen, die unterschiedlich erhaben relativ zueinander sind, quasi gegeneinander bzw. untereinander geschoben werden können, letztlich also näherungsweise ziehharmonikaartig gegeneinander verschoben werden können. Dieses gegeneinander Verschieben verhindert somit eine nennenswerte Faltenbildung, nachdem das Gestrick in definierter Weise aufgrund der mäanderförmigen Rippenstruktur quasi axial zusammengeschoben werden kann und die einzelnen Rippenebenen relativ zueinander verschoben und sogar etwas untereinander geschoben werden können. Eine Faltenbildung wird demzufolge vermieden. Wird das Gelenk wieder gestreckt, so werden, nachdem das Gestrickteil Körperbereiche vor und hinter dem Gelenk hinreichend fest umschließt, insbesondere im Falle eines Kompressionsgestricks, das Gestrick auch im Strukturbereich automatisch wieder gedehnt, mithin also die zuvor deformierten Bereiche in dem Strukturabschnitt aktiv axial wieder auseinandergezogen, so dass die Grundform wieder eingenommen wird.

Befindet sich der Strukturabschnitt auch auf der gegenüberliegenden Gelenkseite, läuft er also beispielsweise komplett um das Gestrickteil herum, so wird der Strukturabschnitt an dieser Seite gedehnt. Hierbei baut der Strukturabschnitt eine inhärente Rückstellkraft auf. Werden die mäandernd verlaufenden Maschenreihen auseinandergezogen, so werden sie relativ zueinander verdehnt, wobei diese Dehnung die Rückstellkraft aufbaut. Wird nun das Gelenk wieder gestreckt, so werden einerseits die Maschen über diese Streckbewegungen wieder zusammengeschoben, andererseits wird diese Bewegung aber auch durch den quasi relaxierenden Strukturabschnitt unterstützt.

Um eine solche mäandernde Rippenstruktur in der Maschenreihenabfolge auszubilden, ist das vorstehend beschriebene Verfahren vorgesehen. Dieses Verfahren sieht zunächst vor, dass im Schritt a) wenigstens eine Maschenreihe mit einer rechts-rechts-Bindung auf beiden Nadelbetten der Strickmaschine mit dem Gestrickfaden gestrickt wird. Bei der Strickmaschine kann es sich bevorzugt um eine Maschine mit einer Maschentransfereinrichtung, insbesondere eine Flachstrickmaschine mit zwei Nadelbetten, nämlich einem ersten und einem zweiten Nadelbett, oft auch als vorderes und hinteres Nadelbett bezeichnet, handeln, auf welcher Maschine eine automatische Herstellung des Gestricks erfolgt, d.h. die Schritte des Rapports werden automatisiert durchgeführt. Grundsätzlich denkbar wäre aber auch ein entsprechender Verfahrensablauf auf einer zwei Nadelbetten aufweisenden Rundstrickmaschine. Unabhängig davon wird in jedem Fall mindestens eine Maschenreihe mit rechts-rechts-Bindung auf beiden Nadelbetten gestrickt.

Sodann wird der elastische Schussfaden eingelegt, wobei dieser elastische Schussfaden zwischen die Platinenmaschen, also zwischen die auf den beiden Nadelbetten befindlichen rechts-rechts-Maschen gelegt wird. Bereits an dieser Stelle der Hinweis, dass die Schritte a) und b) auch gleichzeitig durchgeführt werden können, das heißt, dass zusammen mit dem Stricken der rechts-rechts-Maschen auf beiden Nadelbetten auch der Schussfaden eingelegt werden kann. Im nächsten Schritt c) werden die Maschen auf dem ersten Nadelbett mit dem Gestrickfaden einer rechts-links-Bindung abgestrickt, das heißt, dass hierbei nur auf dem ersten Nadelbett gestrickt wird, beispielsweise dem vorderen Nadelbett, während auf dem zweiten, dann dem hinteren Nadelbett, nicht gestrickt wird. Durch das Abstricken sind demzufolge die Maschen auf dem ersten Nadelbett komplett. Auch dieser Schritt kann gleichzeitig mit den Schritten a) und b), oder auch nur mit dem Schritt b) durchgeführt werden.

Sodann werden im Schritt d) die auf dem ersten Nadelbett befindlichen, abgestrickten Maschen auf das zweite Nadelbett umgehängt. Das heißt, dass beispielsweise die auf dem vorderen Nadelbett gestrickten Maschen auf das hintere Nadelbett gehängt werden. Es werden also alle Maschen auf dem zweiten Nadelbett gesammelt.

Im folgenden Schritt e) werden die Maschen auf dem zweiten Nadelbett nun ebenfalls mit einer rechts-links-Bindung abgestrickt.

Die Schritte a) ― e) bilden den ersten Teil des Rapports bestehend aus den Schritten a) ― j). Hieran schließt sich nun der zweite Teil des Rapports mit den Schritten f) ― j) an.

Es erfolgt nun im nächsten Schritt f) erneut ein Stricken wenigstens einer Maschenreihe mit rechts-rechts-Bindung auf beiden Nadelbetten mit dem Gestrickfaden, quasi entsprechend dem Schritt a).

Sodann wird gemäß Schritt g) wiederum der elastische Schussfaden eingelegt, das heißt, er wird zwischen die Platinenmaschen bzw. zwischen die auf beiden Nadelbetten befindlichen rechts-rechts-Maschen eingelegt. Auch hier bereits der Hinweis, dass dieses Einlegen des elastischen Schussfadens simultan mit dem Stricken der rechts-rechts-Maschen auf beiden Nadelbetten gemäß Schritt f) erfolgen kann.

Im folgenden Schritt h) werden nun die Maschen auf dem zweiten Nadelbett mit dem Gestrickfaden einer rechts-links-Bindung abgestrickt. Hier werden nun also die Reihen auf dem zweiten Nadelbett abgestrickt, während auf dem ersten Nadelbett nichts gestrickt wird. Das Abstricken erfolgt hier also auf dem anderen Nadelbett, verglichen mit dem Schritt c). Nach dem Schritt h) sind demzufolge die Maschen auf dem zweiten Nadelbett komplett. Auch innerhalb dieses Rapportteils können die Schritte f), g) und h) gleichzeitig durchgeführt werden, es können aber auch nur zwei von ihnen gleichzeitig erfolgen.

Im Schritt i) erfolgt nun ein Umhängen der auf dem zweiten Nadelbett befindlichen Maschen auf das erste Nadelbett. In diesem Schritt werden nun alle Maschen auf dem ersten Nadelbett gesammelt, also beispielsweise auf dem vorderen Nadelbett, während im Schritt d) im ersten Rapportteil die Nadeln auf dem zweiten oder hinteren Nadelbett gesammelt wurden.

Schließlich erfolgt im Schritt j) ein Abstricken der auf dem ersten Nadelbett befindlichen Maschen mit dem Gestrickfaden mit einer rechts-links-Bindung.

Mit dem Schritt j) ist nun der zweite Rapportteil abgeschlossen. Mit einem Rapport werden demzufolge zwei, bezogen auf die Dicke des Gestricks versetzt zueinander positionierte respektive in zwei unterschiedlichen Ebenen befindliche Maschenreihen, die natürlich miteinander verbunden sind, gestrickt. Um den Strukturabschnitt in Richtung der Maschenstäbchen, also in Gestricklängsrichtung gesehen, noch zu vergrößern, werden nun die Schritte a) ― j) beliebig oft wiederholt, so dass sich eine fortgesetzte mäanderförmige Maschenreihenrespektive Rippenstruktur ergibt.

Dadurch, dass die beiden abwechselnd gestrickten Maschenreihen respektive Rippen in versetzten Ebenen zueinander liegen, jedoch über den Gestrickfaden natürlich verbunden sind, ist es möglich, die einzelnen Reihen relativ zueinander zu verschieben und sogar quasi leicht untereinander zu schieben. Der Strukturabschnitt folgt demzufolge der geometrischen Veränderung des Gelenks, indem er quasi gestaucht wird und die Maschenreihen zusammengeschoben werden. Beim Dehnen an der gegenüberliegenden Gelenkseite werden die Maschenreihen auseinandergezogen, jedoch baut sich, nachdem sie wie beschrieben miteinander verbunden sind, innerhalb des Gestricks eine Rückstellkraft auf, die die Maschenreihen nach Entlastung quasi wieder zusammenzieht.

Wie bereits beschrieben, besteht die Möglichkeit, dass zumindest zwei der Schritte a), b) und c) sowie f), g) und h) gleichzeitig durchgeführt werden. D.h. es besteht die Möglichkeit, die Schrittfolgen a) und b), b) und c) oder a), b; und c) respektive f) und g), g) und h) oder f), g) und h) gleichzeitig durchzuführen. Die Schritte können aber auch nacheinander durchgeführt werden.

Grundsätzlich besteht die Möglichkeit, das Gestrickteil vollständig als strukturiertes, aus mäandernden Maschenreihen respektive Rippen bestehendes Gestrickteil auszuführen. Alternativ dazu kann auch vorgesehen sein, dass vor Durchführung des ersten Schritts a) eine oder mehrere mit dem Gestrickfaden gestrickte Grundgestrickmaschenreihen in einer Grundgestrickbindungsart mit eingelegtem Schussfaden gestrickt werden, wie auch die Möglichkeit besteht, nach Durchführung des letzten Schritts j) eine oder mehrere mit dem Gestrickfaden gestrickte Grundgestrickmaschenreihen in einer Grundgestrickbindungsart mit eingelegtem Schussfaden zu stricken. Das heißt, dass vor und/oder nach dem Strukturabschnitt, gesehen in Richtung der Maschenstäbchen, entsprechende Grundgestrickabschnitte gestrickt werden können, mithin also der Strukturabschnitt quasi in das Grundgestrick integriert eingestrickt wird. Die Grundgestrickbindungsart kann beliebig sein, beispielsweise eine rechts-rechts-Bindung, eine rechts-links-Bindung etc., wobei die Grundgestrickbindungsarten in den in Maschenstäbchenrichtung versetzten Grundgestrickbereichen gleich oder unterschiedlich sein können.

In weiterer Ausgestaltung des erfindungsgemäßen Verfahrens ist es möglich, dass die in den Schritten a) ― j) gestrickten Maschenreihen über die gesamte Breite des Gestrickteils gestrickt werden. Da eine Bandage oder ein Bein- oder Armstrumpf ein schlauchförmiges Gestrickteil sind, erstreckt sich demzufolge der mäandernde Strukturabschnitt um 360° um das gesamte Gestrickteil.

Alternativ dazu besteht die Möglichkeit, dass die in den Schritten a) ― j) gestrickten Maschenreihen nur abschnittsweise über die Breite des Gestrickteils gestrickt werden, wobei gesehen in Richtung der Gestrickbreite (also senkrecht zu den Maschenstäbchen) an einer oder an beiden Seiten der in den Schritten a) ― j) gestrickten Maschenreihen sich Grundgestrickmaschenreihen mit der Grundgestrickbindungsart anschließen. Gemäß dieser Erfindungsausgestaltung wird also der mäandernde Strukturabschnitt quasi in das Grundgestrick eingebettet, dass rechts und/oder links, gesehen in Richtung der Gestrickbreite, gestrickt werden kann und natürlich auch darüber und/oder darunter. Es sind also beliebige Einbindungs- oder Positionierungsmöglichkeiten bezüglich des Strukturteils denkbar, wie natürlich auch mehrere separate Strukturabschnitte an verschiedenen Positionen eingebunden werden können.

In weiterer Erfindungsausgestaltung kann vorgesehen sein, dass zur Ausbildung eines oder mehrerer Spickel in den Schritten a) ― j) gestrickte Maschenreihen gesehen in Richtung der Gestrickbreite nur abschnittsweise und mit variierender Reihenlänge gestrickt werden, wobei benachbart zu jeder oder zu mehreren kürzeren Maschenreihen eine über die gesamte Breite durchgehende Maschenreihe gestrickt wird. Gemäß dieser Erfindungsausgestaltung wird demzufolge ein oder werden mehrere Spickel eingestrickt, wobei diese Spickel, verglichen mit einer maximalen Maschenreihenlänge, deutlich kürzer sind. Diese Maschenreihen werden quasi zwischen zwei längeren Maschenreihen gestrickt, so dass das Gestrick aufgrund der zusätzlichen kurzen Maschenreihen quasi geometrisch geformt werden kann und es möglich ist, ihm eine, beispielsweise abweichend von einer reinen Schlauchform, etwas andere Form zu verleihen. Es besteht dabei die Möglichkeit, eine kürzere Maschenreihe zwischen zwei längere durchgehende Maschenreihen zu stricken, alternativ dazu können auch mehrere kürzere Maschenreihen zwischen zwei durchgehende gestrickt werden. Ein solcher Spickel kann nur an einer Längsseite des Gestrickteils bzw. Strukturteils, sich in Breitenrichtung zur Gestrickmitte hin erstreckend, vorgesehen sein, alternativ kann auch an einer gegenüberliegenden Längsseite des Gestrick- bzw. Strukturteils ein Spickel vorgesehen sein, wobei diese Spickel aber auch, gesehen in Richtung der Maschenstäbchen, axial versetzt zueinander sein können. Weiterhin ist es denkbar, mit zwei oder mehreren unterschiedlichen Farben aufweisenden Gestrickfäden zu stricken. Dies ermöglicht es, dem Strukturabschnitt, gegebenenfalls auch dem oder den benachbarten Grundgestrickbereichen, ein optisch sehr ansprechendes, farblich variierendes Äußeres zu verleihen. Beispielsweise ist es denkbar, im ersten Rapportteil einen estrickfaden mit einer ersten Farbe und im zweiten Rapportteil einen Gestrickfaden mit einer zweiten Farbe zu verwenden, so dass sich letztlich quasi farblich unterscheidende mäandernde Maschenreihen gestrickt werden. Das heißt, dass auf den beiden Nadelbetten mit unterschiedlich farbigen Gestrickfäden gestrickt wird oder Ähnliches, die Variationsmöglichkeiten sind hier mannigfaltig. Auch können Gestrickfäden mit unterschiedlichen Faden- oder Garnqualitäten oder Gestrickfäden, die aus unterschiedlichen Faden- oder Garnmaterialien bestehen, verwendet werden, die in dem Strukturabschnitt oder auch dem oder den benachbarten Gestrickbereichen verstrickt werden. Z.B. können der Strukturabschnitt und der oder die benachbarten Gestrickabschnitten aus Fäden mit unterschiedlichen Qualitäten und/oder Materialien gestrickt werden, so dass sich die Bereiche diesbezüglich unterscheiden. Alternativ können in der Qualität und/oder im Material unterschiedliche Fäden auch innerhalb des Strukturabschnitts oder einem benachbarten Gestrickbereich oder in beiden verstrickt werden. Auch hier besteht folglich eine große Variationsmöglichkeit.

Als Strickmaschine wird besonders bevorzugt eine Maschine mit einer Maschentransfereinrichtung verwendet, insbesondere eine Flachstrickmaschine mit einem ersten und einem zweiten respektive einem vorderen und einem hinteren Nadelbett verwendet, so dass das Gestrickteil ein Flachgestrick ist, das über eine Längsnaht zur Schlauchform, der Bandagen- oder Beinstrumpf- oder Armstrumpfform entsprechend vernäht wird.

Besonders bevorzugt handelt es sich bei dem Gestrickteil um ein kompressives Gestrickteil, das, wie einleitend beschrieben, einen bestimmten Druck auf das übergriffene Gewebe ausübt, wobei dieser Druck zu medizinischen oder therapeutischen Zwecken ausgeübt wird. Wie beschrieben unterscheidet sich ein solches kompressives Strickteil von einem üblichen, eine gewisse Grundelastizität aufweisenden Gestrickteil dadurch, dass ein bestimmter hoher Mindestdruck, wie er in der einleitend bereits beschriebenen Güte- und Prüfbestimmung beispielsweise für medizinische Kompressionsstrümpfe definiert ist, aufweist, wobei hierunter auch unterschiedliche Kompressionsklassen unterschieden werden. Üblicherweise werden die Klassen I ― IV unterschieden, wie auch in der genannten Gütesicherung RAL GZ 387/1 der Fall ist. Wenngleich diese Güte- und Prüfbestimmungen den Bereich medizinischer Kompressionsstrümpfe betreffen, ist diese RAL in entsprechender Weise beispielsweise auch auf die Ausbildung von Armstürmpfen oder Bandagen oder Orthesen oder dergleichen anzuwenden.

Neben dem erfindungsgemäßen Verfahren betrifft die Erfindung ferner ein Gestrickteil, das verfahrensgemäß hergestellt ist. Dieses Gestrickteil weist, wie vorstehend ausgeführt, einen entsprechenden Strukturabschnitt mit den einander mäanderförmig abwechselnden Maschenreihen, die in unterschiedlichen Ebenen liegen, auf, wobei die Gestrickteilgeometrie allein durch das erfindungsgemäße Verfahren respektive die Abfolge der einzelnen Schritte erzeugt und definiert wird.

Daneben betrifft die Erfindung ein Gestrickteil, bestehend aus wenigstens einem Gestrickfaden und wenigstens einem elastischen Schussfaden, umfassend wenigstens einen Strukturabschnitt bestehend aus mehreren parallel zueinander liegenden, gesehen in Gestricklängsrichtung mäanderförmig verlaufenden Maschenreihen, die bei Einwirken einer in Längsrichtung des Gestricks wirkenden Zugbeanspruchung unter Aufbau einer elastischen Rückstellkraft auseinanderziehbar sind, und die bei Einwirken einer entgegengesetzt wirkenden Druckbeanspruchung gegeneinander verschiebbar sind. Gestricklängsrichtung bedeutet in diesem Zusammenhang in Richtung der Maschenstäbchen.

Der Struckturabschnitt kann sich über die gesamte Breite des Gestrickteils oder nur abschnittsweise über die Breite des Gestrickteils erstrecken, das heißt, er kann bei einem schlauchförmigen Gestrickteil entweder um 360° umlaufen, oder nur um einen Teilabschnitt, beispielsweise 270° oder 180° oder weniger. Die Breite kann beliebig gewählt werden, je nach Typ des Gestrickteils und je nach Tragort am Körper etc.

Des Weiteren kann im Strukturteil wenigstens ein Spickel gestrickt werden, wobei natürlich auch die Ausbildung mehrerer Spickel denkbar ist. Die Spickel werden durch Einstricken respektive Ausbilden mehrerer kürzerer Maschenreihen, verglichen mit den übrigen Maschenreihen mit größerer oder maximaler Länge, erzeugt, wie dem Fachmann hinreichend bekannt.

Weiterhin ist es denkbar, dass das Gestrickteil aus wenigstens zwei unterschiedliche Farben aufweisenden Gestrickfäden gestrickt ist, wobei die Verwendung dieser unterschiedlich farbigen Gestrickfäden letztlich beliebig ist, also ihre Verarbeitung auf dem ersten oder dem zweiten Nadelbett letztlich beliebig gewählt werden kann, so dass demzufolge dem Gestrickteil ein beliebiges farbiges Erscheinungsbild verliehen werden kann. Auch können Gestrickfäden mit unterschiedlichen Faden- oder Garnqualitäten oder Gestrickfäden, die aus unterschiedlichen Faden- oder Garnmaterialien bestehen, verwendet werden, die in dem Strukturabschnitt oder auch dem oder den benachbarten Gestrickbereichen verstrickt werden.

Weitere Vorteile und Einzelheiten der vorliegenden Erfindung ergeben sich aus den im Folgenden beschriebenen Ausführungsbeispielen sowie anhand der Zeichnungen. Dabei zeigen:
- Fig. 1: eine Prinzipdarstellung zur Erläuterung des erfindungsgemäßen Verfahrens anhand einer Darstellung des Rapports,
- Fig. 2: eine Prinzipdarstellung des Strukturbereichs im entspannten Zustand,
- Fig. 3: eine Prinzipdarstellung des Strukturbereichs im gestauchten Zustand,
- Fig. 4: eine Prinzipdarstellung des Strukturbereichs im gezogenen Zustand,
- Fig. 5: eine Ansicht einer Seite des Strukturabschnitts in einem Ausschnitt,
- Fig. 6: eine Prinzipdarstellung eines ersten Gestrickteils,
- Fig. 7: eine Prinzipdarstellung eines zweiten Gestrickteils,
- Fig. 8: eine Prinzipdarstellung eines Gestrickteils einer dritten Ausführungsform mit Blick auf den im Bereich der Kniebeuge anzuordnenden Strukturabschnitt, und
- Fig. 9: das Gestrickteil aus Fig. 8 von der anderen Seite.

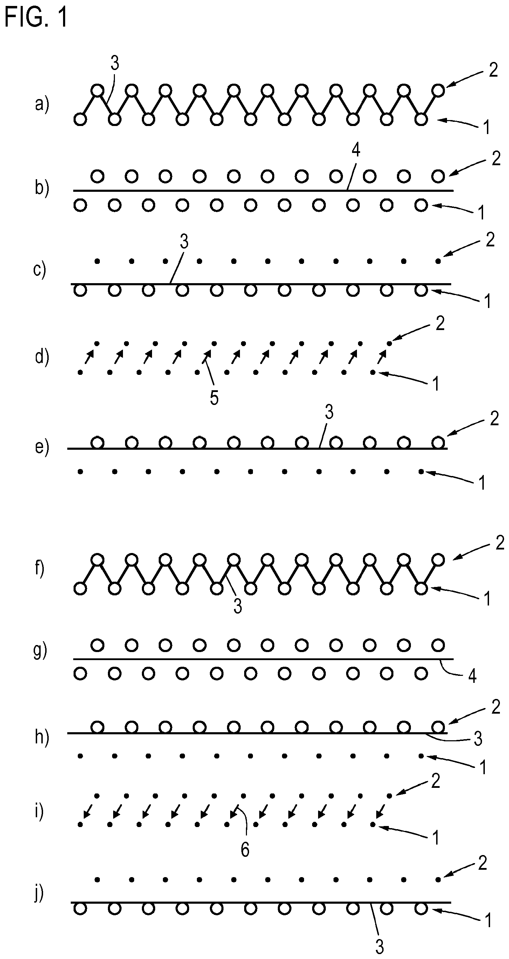

Fig. 1 zeigt in Form einer Prinzipdarstellung einen vollständigen Rapport, mit dem ein Gestrickteil mit einem erfindungsgemäßen Strukturabschnitt, bevorzugt auf einer Strickmaschine mit einer Maschentransfereinrichtung, insbesondere einer Flachstrickmaschine, gebildet werden kann. Gezeigt sind die Schritte a) ― j), die der Reihe nach durchgeführt werden können, wobei die Schritte a) und b) respektive f) und g) auch simultan durchgeführt werden können.

Vor Beginn des Rapports besteht die Möglichkeit, über eine beliebige Länge mit wenigstens einem oder dem Gestrickfaden einen entsprechenden Grundgestrickbereich zu stricken, mit einer beliebigen Grundgestrickbindung. Dies, wie auch die Schritte a) ― j), werden bevorzugt auf einer Flachstrickmaschine mit einem ersten und einem zweiten Nadelbett durchgeführt.

Der Rapport selbst beginnt im Schritt a) damit, dass wenigstens eine Maschenreihe mit rechts-rechts-Bindung auf dem ersten Nadelbett 1 und auf dem zweiten Nadelbett 2 unter Verwendung eines Gestrickfadens 3 gestrickt werden.

Im Schritt b) wird, entweder nachfolgend oder simultan zum Schritt a), ein elastischer Schussfaden 4 zwischen die Platinenmaschen, also zwischen die im Schritt a) auf dem ersten und dem zweiten Nadelbett 1, 2 gestrickten Maschen eingelegt. Der Begriff "Platinenmasche" beschreibt den Bereich zwischen den auf den beiden Nadelbetten 1, 2 gestrickten Maschen.

Im Schritt c) werden, gegebenenfalls simultan mit dem Schritt b) oder den Schritten a) und b), die Maschen auf dem ersten Nadelbett 1 mit dem Gestrickfaden 3 abgestrickt. Dies erfolgt in einer rechts-links-Bindung. Wie in Fig. 1 gezeigt, wird auf dem hinteren zweiten Nadelbett 2 nicht gestrickt.

Im nächsten Schritt d) werden, wie durch die Pfeile 5 gezeigt, die abgestrickten Maschen des ersten Nadelbetts 1 auf die Nadeln des zweiten Nadelbetts 2 umgehängt. Das heißt, dass alle Maschen auf dem zweiten Nadelbett 2 respektive auf dessen Nadeln gesammelt werden. Auf diesem befinden sich somit die im Schritt a) auf dem zweiten Maschenbett gestrickten Maschen sowie die im Schritt c) abgestrickten Maschen des ersten Nadelbetts 1.

Nach dem Umhängen wird gemäß Schritt e) mit dem Gestrickfaden 3 noch auf dem zweiten Nadelbett 2 abgestrickt, während auf dem ersten Nadelbett 1 nicht gestrickt wird. Mit dem Schritt e) endet der erste Rapportteil umfassend die Schritte a) ― e).

Der zweite Rapportteil umfasst die Schritte f) ― j). Gemäß Schritt f) wird nun, entsprechend Schritt a), auf dem ersten und zweiten Nadelbett 1, 2 mit dem Gestrickfaden 3 mit rechts-rechts-Bindung gestrickt. Es wird also auch hier wiederum auf beiden Nadelbetten gestrickt.

Im Schritt g) erfolgt, entsprechend Schritt b), das Einlegen des elastischen Schussfadens 4 zwischen die Platinenmaschen, also zwischen die auf den Nadelbetten 1, 2 gestrickten Maschen. Auch dieser Schritt kann simultan zum Schritt f) durchgeführt werden.

Im Schritt h) werden nun ― anders als im Schritt c) ― die Maschen auf dem zweiten Nadelbett 2 respektive auf dessen Nadeln mit dem Gestrickfaden 3 abgestrickt. Auf dem ersten Nadelbett 1 wird nicht gestrickt. Schritt h) kann simultan mit Schritt g) oder den Schritten f) und g) durchgeführt werden.

Im Schritt i) werden nun die abgestrickten Maschen des zweiten Nadelbetts 2 auf die Nadeln des ersten Nadelbetts 1 umgehängt, wie durch die Pfeile 6 dargestellt ist. Die Umhängerichtung ist hier also entgegengesetzt zu der gemäß Schritt d). Im Schritt i) werden also die Maschen auf den Nadeln des vorderen ersten Nadelbetts 1 gesammelt, das nun die im Schritt f) hierauf gestrickten Maschen wie auch die abgestrickten Maschen des zweiten Nadelbetts aufweist.

Sodann wird im Schritt j) mit dem Gestrickfaden 3 auf dem ersten Nadelbett 1 abgestrickt.

Sodann beginnt der Rapport mit den Schritten a) ― j) erneut. Der Rapport wird so lange durchgeführt, wie der hierüber hergestellte Strukturabschnitt lang sein soll, gesehen in Richtung der Maschenstäbchen.

Ähnlich wie vor Durchführung des ersten Rapports ist es nach Durchführung des letzten Rapports denkbar, erneut unter Verwendung des wenigstens einen Gestrickfadens 3 und des Schussfadens 4 ein Grundgestrick weiter zu stricken, wobei auch dieses Grundgestrick, ähnlich wie auch das gegebenenfalls zu Beginn gestrickte Grundgestrick, mit einer Grundgestrickbindungsart beliebiger Strickweise, beispielsweise ein reines rechts-rechts-Gestrick oder ein rechts-links-Gestrick oder dergleichen, sein kann. Es sind beliebige Grundgestrickarten denkbar.

In Querrichtung des Gestrickteils respektive in Richtung der Breite, also senkrecht zu den Maschenstäbchen, können die einzelnen Maschenreihen entweder über die gesamte Breite gestrickt werden, oder nur abschnittsweise, worauf nachfolgend noch eingegangen wird.

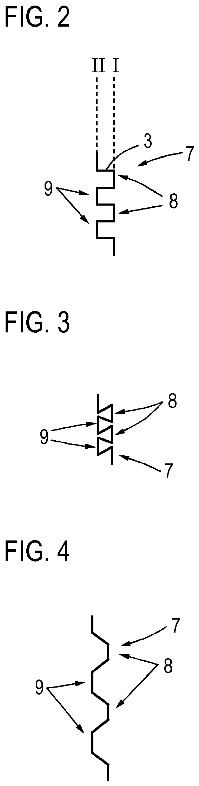

Die Figuren 2 ― 4 zeigen Prinzipdarstellungen des über die Schritte a) ― j) gestrickten Strukturbereichs zur Erläuterung seiner Struktur respektive Geometrie und der Funktion. Die Figuren zeigen jeweils Schnitt- oder Seitenansichten.

Fig. 2 zeigt den Strukturbereich 7 im entspannten, normalen Zustand. Gezeigt sind mäandernde Maschenreihen 8, 9, die auf den jeweiligen beiden Nadelbetten 1, 2 mit Hilfe des erfindungsgemäßen Verfahrens gestrickt wurden. Beispielsweise werden die Maschenreihen 8 über den ersten Rapportteil umfassend die Schritte a) ― e) auf dem ersten Nadelbett 1 gebildet, während die Maschenreihen 9 über den zweiten Rapportteil umfassend die Schritte f) ― j) auf den Nadeln des zweiten Nadelbetts 2 gebildet werden.

Ersichtlich sind die einzelnen Maschenreihen 8, 9 natürlich über den Gestrickfaden 3 miteinander verbunden, sie befinden sich jedoch auf unterschiedlichen Ebenen I, II, wie in Fig. 2 dem Grunde nach gezeigt. Es bildet sich also eine Mäanderstruktur aus. Aus Gründen der Einfachheit ist der jeweilige durchlaufende eingelegte elastische Schussfaden hier nicht gezeigt.

Der gestrickte Strukturabschnitt 7 wird in der Tragstellung im Bereich einer Gelenkbeuge, beispielsweise eines Knie- oder Armgelenks, angeordnet. Wird das Bein oder der Arm angewinkelt, so wird der Strukturbereich in der Gelenkbeuge zusammengeschoben und gestaucht, was in Fig. 3 gezeigt ist. Aufgrund der Mäanderstruktur kommt es quasi zu einem ziehharmonikaartigen Zusammenschieben, im Rahmen dessen die Maschenreihen 8, 9 gegeneinander und auch etwas untereinander geschoben werden, nachdem sie sich wie ausgeführt auf unterschiedlichen Ebenen I, II befinden. Es wird also eine Geometrieänderung eingeleitet, indem der Strukturabschnitt lediglich zusammengeschoben wird, ohne dass hierbei ein nennenswerter Faltenwurf resultierend aus einem Zusammenschieben des Materials an sich erfolgt. Dies ist für den Träger sehr angenehm, denn eine Faltenbildung ist störend. Beim erneuten Strecken des Gelenks wird der gestauchte Zustand auch wieder aufgelöst, nachdem das Gestrickteil einerseits aufgrund seiner hinreichend festen Auflage an den benachbarten Körperpartien mitgenommen wird, zum anderen schiebt sich der gestauchte Strukturabschnitt 7, der gegebenenfalls eine aus dem Stauchen resultierende geringe Rückstellkraft aufbaut, auch wieder auseinander und geht in den entspannten Normalzustand gemäß Fig. 2 über. Es findet demzufolge im Gelenk sowie oberhalb und unterhalb des Gelenks keine Faltenbildung statt respektive wird extrem verringert, auch wenn das Gelenk mehrfach gebeugt wird. Ebenso kommt es zu einer Minderung des Drucks in der Gelenkbeuge beim Anwinkeln, da das cE-Maß beim Anwinkeln zunimmt.

Fig. 4 zeigt einen Strukturabschnittszustand, bei dem der Strukturabschnitt 7 gedehnt wird. Dies ist der Fall, wenn der Strukturabschnitt beispielsweise am Ellenbogen- oder Kniebereich positioniert ist. Hierbei werden die Maschenreihen 8, 9 in Gestricklängsrichtung, also in Richtung der Maschenstäbchen auseinandergezogen, das heißt, dass die Mäanderstruktur quasi in Längsrichtung gedehnt wird. Fig. 4 zeigt den gespannten Zustand. Ersichtlich befinden sich hierbei die einzelnen Maschenreihen 8, 9 nach wie vor leicht ebenenmäßig versetzt. Infolge der Längsdehnung baut sich eine inhärente Rückstellkraft auf, die sich auch in jedoch geringerem Maße bei der Stauchung gemäß Fig. 3 aufbaut. Diese relativ hohe Rückstellkraft führt dazu, dass sich das Gestrickteil respektive der Strukturabschnitt, wenn das Gelenk wieder gestreckt und entspannt wird, automatisch zusammenzieht und in den entspannten Zustand gemäß Fig. 2 übergeht.

Insgesamt ermöglicht somit die links-links-Bindung mit eingelegtem Schussfaden die Ausbildung der erfindungsgemäßen Rippenstruktur und damit des Strukturbereichs 7 mit den vorstehend beschriebenen Eigenschaften und Vorteilen.

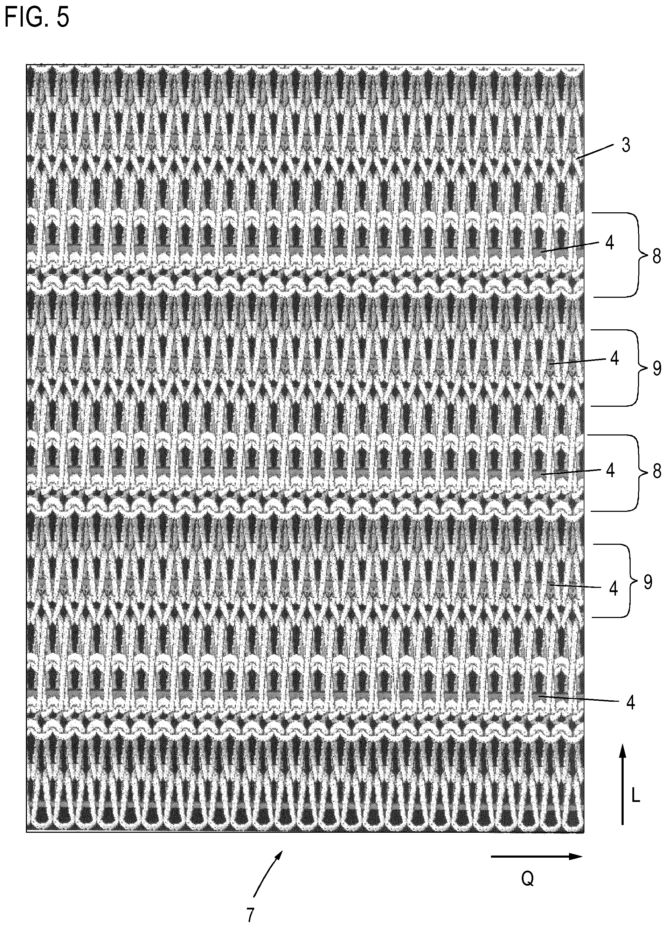

Fig. 5 zeigt eine Aufsicht auf den Strukturabschnitt 7 in etwas gedehntem Zustand, so dass die einzelnen Abschnitte gut sichtbar sind.

Gezeigt sind exemplarisch zwei Maschenreihen 8 sowie zwei Maschenreihen 9, wobei die beiden Maschenreihen 8 zur Oberseite hin erhaben sind, während die Maschenreihen 9 zur Unterseite hin erhaben sind. Die einzelnen Maschenreihen sind über entsprechende Verbindungen seitens des Gestrickfadens 3 natürlich verbunden. Dargestellt ist des Weiteren auch der eingelegte elastische Schussfaden 4, der in den jeweils erhabenen Maschenreihen 8, 9 verläuft.

Mit dem Pfeil L ist auch die Gestricklängsrichtung, also die Richtung der Maschenstäbchen, und mit dem Pfeil Q die Gestrickquerrichtung, also die Breitenrichtung senkrecht zu den Maschenstäbchen angegeben.

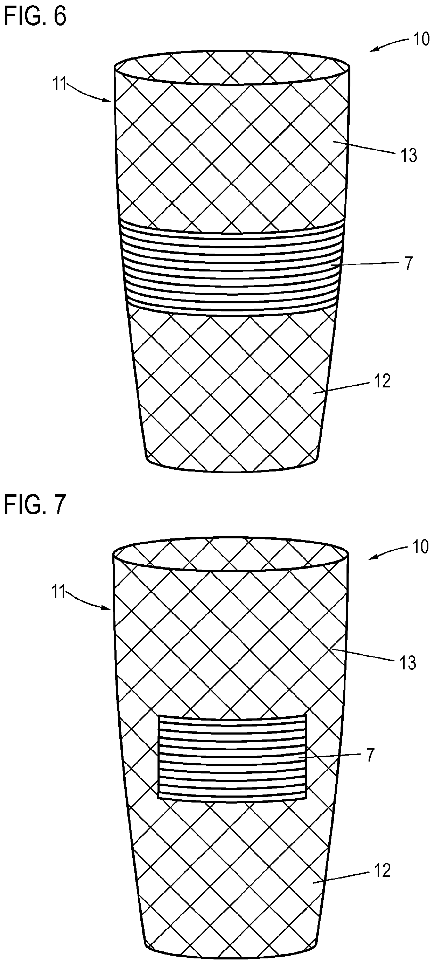

Fig. 6 zeigt ein erstes Beispiel eines erfindungsgemäßen Gestrickteils 10 hier in Form einer Kniebandage 11. Es handelt sich um ein schlauchförmiges Gestrickteil 10, das auf einer Flachstrickmaschine mit zwei Nadelbetten gestrickt und sodann über eine Längsnaht zu der Schlauchform verbunden ist.

Das Gestrickteil 10 weist hier drei unterscheidbare Gestrickbereiche auf, nämlich einen ersten Grundgestrickbereich 12, der aus dem Gestrickfaden 3 mit eingelegtem Schussfaden 4 in einer Grundgestrickbindung beliebiger Art gestrickt ist. Diesem ersten Grundgestrickbereich 12 folgt der Strukturabschnitt 7, der in der erfindungsgemäßen Weise wie vorstehend beschrieben gestrickt ist. An diesen schließt sich sodann in Richtung der Maschenstäbchen ein zweiter Grundgestrickbereich 13 aus dem Gestrickfaden 3 mit eingelegtem Schussfaden 4 an, der ebenfalls in einer beliebigen Grundgestrickbindungsart gestrickt ist, die die gleiche sein kann wie im Grundgestrickbereich 12, aber auch eine andere sein kann.

Bei dem Ausführungsbeispiel gemäß Fig. 6 läuft der Strukturabschnitt 7 vollständig um, also um 360° und bildet eine Ringform. Die erhabenen respektive mäandernden Maschenreihen sind durch die Querlinien dargestellt.

Fig. 7 zeigt ein Gestrickteil 10 einer zweiten Ausführungsform wiederum in Form einer Kniebandage 11, bei der ein großer Grundgestrickbereich 12 gestrickt ist, in den der Strukturbereich 7 allseitig eingebettet ist. Das heißt, dass hier die Maschenreihen 8, 9 des Strukturbereichs 7 nur abschnittsweise über die Breite (Q in Fig. 5) des Flachgestricks gestrickt werden. Der Umfangsbereich, um den die Maschenreihen 8, 9 respektive der Strukturbereich 7 gestrickt wird, kann hierbei beliebig variieren, je nach Anwendungsform oder gewünschtem Tragekomfort etc.

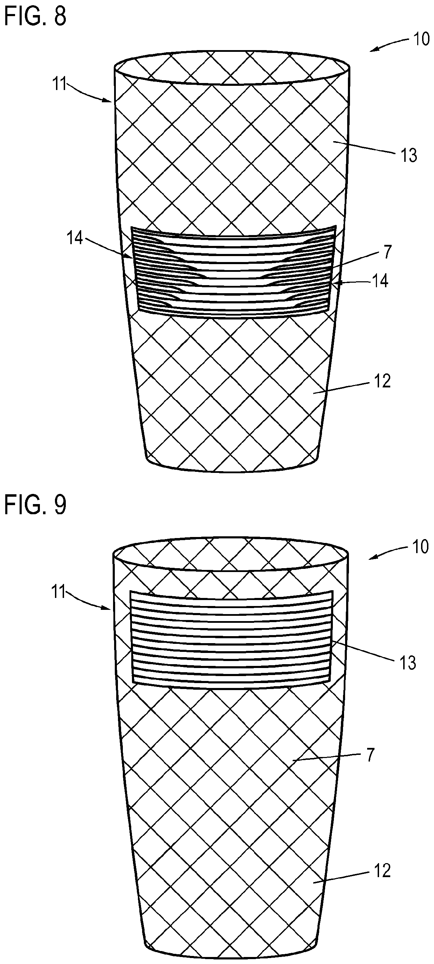

Fig. 8 zeigt eine dritte Ausführungsform eines erfindungsgemäßen Gestrickteils 10 wiederum in Form einer Kniebandage 11. Auch dieses Gestrickteil 10 weist einen Grundgestrickbereich 12 auf, in den ein erster Strukturbereich 7 eingebettet ist, der also vollständig vom Grundgestrickbereich 12 umgeben ist. Dieser Strukturbereich 7 befindet sich in der Tragstellung im Bereich der Kniekehle, das heißt, dass Fig. 8 die Bandagenrückseite zeigt.

Bei dieser Ausgestaltung ist der Strukturbereich 7 beidseits mit jeweils einem Spickel 14 gestrickt, wie durch die über die deutlich kürzeren, nicht durchlaufenden Linien, die kürzere Maschenreihen darstellen, dargestellt ist. Ein solcher Spickel 14 wird durch Stricken kurzer Maschenreihen zwischen durchlaufende Maschenreihen erzeugt, das heißt, dass gesehen in Richtung der Maschenstäbchen (L in Fig. 5) die Maschenreihenanzahl innerhalb der Maschenstäbchen in Querrichtung (Q in Fig. 5) des Gestricks variiert. Dies ermöglicht eine geometrische Formung des gesamten Gestricks.

Fig. 9 zeigt schließlich die Vorderseite des Gestrickteils 10 respektive der Kniebandage 11 aus Fig. 8 mit dem Grundgestrickbereich 12 sowie einem zweiten Strukturabschnitt 7, der auch hier nur abschnittsweise gestrickt ist, also nicht vollständig umläuft, und der spickelfrei gestrickt ist. Er befindet sich exemplarisch weiter oberhalb des an der anderen Seite befindlichen ersten Strukturabschnitts 7 und in der Tragstellung oberhalb der Kniescheibe, die beispielsweise von einer im Gestrickteil 10 integrierten, nicht näher gezeigten Pelotte oder dergleichen eingefasst oder übergriffen oder dergleichen ist.

Beim Bewegen respektive Abwinkeln des Knies wird der erste Strukturbereich 7, der in der Kniekehle angeordnet ist, im Sinne der Fig. 3 gestaucht, die Maschenreihen werden also relativ zueinander zusammengeschoben respektive untereinander geschoben.

Demgegenüber wird der an der anderen Seite befindliche zweite Strukturabschnitt 7 oberhalb der Kniescheibe gedehnt, entsprechend Fig. 4 werden also die Maschenreihen auseinandergezogen. In beiden Fällen baut sich eine Rückstellkraft auf, im Falle des Stauchens natürlich eine deutlich geringere als im Falle der Dehnung.

Beim erneuten Strecken des Beins werden, nachdem die Bandage fest auf dem Unter- und Oberschenkel sitzt, die beiden Strukturbereiche 7 wieder in ihren entspannten Ausgangszustand zurückbewegt, wobei dies im Falle des kniekehlenseitigen Strukturbereichs 7 primär durch die Streckbewegung erfolgt, da dort nur eine relativ geringe Rückstellkraft gegeben ist, während die Rückstellbewegung des im Bereich der Kniescheibe befindlichen Sturkturbereichs 7 über die aufgebaute Rückstellkraft unterstützt wird.

Bei dem Gestrickteil 10 handelt es sich bevorzugt um ein Kompressionsgestrick, das also einen definierten Kompressionsdruck auf das darunterliegende Gewebe ausübt.

Während die Figuren 6 ― 9 eine Kniebandage zeigen, kann das Gestrickteil 10 aber auch anderer Art sein, beispielsweise ein einfacher Beinstrumpf, beispielsweise ein kompressiver Thrombosestrumpf, oder ein Armstrumpf mit kompressiver Eigenschaft, oder eine Orthese oder dergleichen, wobei die Aufzählung nicht hierauf begrenzt ist.

## Patentansprüche

1. Verfahren zur Herstellung eines Gestrickteils (10) aus wenigstens einem Maschen bildenden Gestrickfaden (3) und wenigstens einem elastischen Schussfaden (4) auf einer ein erstes und ein zweites Nadelbett (1, 2) aufweisenden Stickmaschine, mit folgenden Schritten:
a) Stricken wenigstens einer Maschenreihe (8, 9) mit rechts-rechts-Bindung auf beiden Nadelbetten (1, 2) mit dem Gestrickfaden (3),
b) Einlegen des elastischen Schussfadens (4),
c) Abstricken der Maschen auf dem ersten Nadelbett (1) mit dem Gestrickfaden (3) mit einer rechts-links-Bindung,
d) Umhängen der auf dem ersten Nadelbett (1) befindlichen Maschen auf das zweite Nadelbett (2),
e) Abstricken der Maschen auf dem zweiten Nadelbett (2) mit dem Gestrickfaden (3) mit einer rechts-links-Bindung,
f) Stricken wenigstens einer Maschenreihe (8, 9) mit rechts-rechts-Bindung auf beiden Nadelbetten (1, 2) mit dem Gestrickfaden (3),
g) Einlegen des elastischen Schussfadens (4),
h) Abstricken der Maschen auf dem zweiten Nadelbett (2) mit dem Gestrickfaden (3) mit einer rechts-links-Bindung,
i) Umhängen der auf dem zweiten Nadelbett (2) befindlichen Maschen auf das erste Nadelbett (1),
j) Abstricken der Maschen auf dem ersten Nadelbett (1) mit dem Gestrickfaden (3) mit einer rechts-links-Bindung,
k) beliebig häufiges Wiederholen der Schritte a) ― j).

2. Verfahren nach Anspruch 1, **dadurch gekennzeichnet, dass** zumindest zwei der Schritte a), b) und c), und/oder f), g) und h) gleichzeitig durchgeführt werden.

3. Verfahren nach Anspruch 1 oder 2, **dadurch gekennzeichnet, dass** vor Durchführung des ersten Schritts
a) eine oder mehrere mit dem Gestrickfaden (3) gestrickte Grundgestickmaschenreihen in einer Grundgestrickbindungsart, vorzugsweise mit eingelegtem Schussfaden (4), gestrickt werden.

4. Verfahren nach einem der vorangehenden Ansprüche, **dadurch gekennzeichnet, dass** nach Durchführung des letzten Schritts j) eine oder mehrere mit dem Gestrickfaden (3) gestrickte Grundgestickmaschenreihen in einer Grundgestrickbindungsart, vorzugsweise mit eingelegtem Schussfaden (4) gestrickt werden.

5. Verfahren nach einem der vorangehenden Ansprüche, **dadurch gekennzeichnet, dass** die in den Schritten a) ― j) gestrickten Maschenreihen über die gesamte Breite des Gestrickteils (10) gestrickt werden.

6. Verfahren nach einem der Ansprüche 1 bis 4, **dadurch gekennzeichnet, dass** die in den Schritten a) ― j) gestrickten Maschenreihen nur abschnittsweise über die Breite des Gestrickteils (10) gestrickt werden, wobei gesehen in Richtung der Gestrickbreite an einer oder an beiden Seiten der in den Schritten a) ― j) gestrickten Maschenreihen sich Grundgestrickmaschenreihen mit der Grundgestrickbindungsart anschließen.

7. Verfahren nach einem der vorangehenden Ansprüche, **dadurch gekennzeichnet, dass** zur Ausbildung eines oder mehrerer Spickel (14) in den Schritten a) ― j) gestrickte Maschenreihen gesehen in Richtung der Gestrickbreite nur abschnittsweise mit variierender Reihenlänge gestrickt werden, wobei benachbart zu jeder oder zu mehreren kürzeren Maschenreihen eine über die gesamte Breite durchgehende Maschenreihe gestrickt wird.

8. Verfahren nach einem der vorangehenden Ansprüche, **dadurch gekennzeichnet, dass** mit zwei oder mehreren unterschiedliche Farben, Fadenqualitäten und/oder Fadenmaterialien aufweisenden Gestrickfäden (3) gestrickt wird.

9. Verfahren nach einem der vorangehenden Ansprüche, **dadurch gekennzeichnet, dass** als Strickmaschine eine Maschine mit einer Maschentransfereinrichtung verwendet wird.

10. Gestrickteil bestehend aus wenigstens einem Gestrickfaden (3) und wenigstens einem elastischen Schussfaden (4), umfassend wenigstens einen aus dem Gestrickfaden (3) gestrickten Strukturabschnitt (7) **dadurch gekennzeichnet dass** der Strukturabschnitt (7) aus mehreren parallel zueinander liegenden, gesehen in Gestricklängsrichtung (L) mäanderförmig verlaufenden, zur Oberseite und zur Unterseite des Strukturabschnitts (7) erhabenen Maschenreihen besteht, in die der Schussfaden (4) eingelegt ist, und die bei Einwirken einer in Längsrichtung (L) des Gestrickteils (10) wirkenden Zugbeanspruchung unter Aufbau einer elastischen Rückstellkraft auseinanderziehbar sind, und die bei Einwirken einer entgegengesetzt wirkenden Druckbeanspruchung gegeneinander verschiebbar sind.

11. Gestrickteil nach Anspruch 10, **dadurch gekennzeichnet, dass** sich der Strukturabschnitt (7), gesehen in Gestricklängsrichtung (L), an wenigstens einen vor- oder nachgeschalteten Grundgestrickabschnitt (12, 13) anschließt.

12. Gestrickteil nach Anspruch 10 oder 11, **dadurch gekennzeichnet, dass** sich der Strukturabschnitt (7) über die gesamte Breite (Q) des Gestrickteils (10) oder nur abschnittsweise über die Breite (Q) des Gestrickteils (10) erstreckt.

13. Gestrickteil nach einem der Ansprüche 10 bis 12, **dadurch gekennzeichnet, dass** im Strukturabschnitt (7) wenigstens ein Spickel (14) gestrickt ist.

14. Gestrickteil nach einem der Ansprüche 10 bis 13, **dadurch gekennzeichnet, dass** es aus wenigstens zwei unterschiedliche Farben, Fadenqualitäten und/oder Fadenmaterialien aufweisenden Gestrickfäden (3) gestrickt ist.

## Claims

1. Method for producing a knitted article (10) from at least one stitch-forming knitted fabric thread (3) and at least one elastic weft thread (4) on a knitting machine having a first and a second needle bed (1, 2), having the following steps:
a) knitting at least one stitch course (8, 9) with a right-right pattern on both needle beds (1, 2) with the knitted fabric thread (3),
b) introducing the elastic weft thread (4),
c) casting off the stitches on the first needle bed (1) with the knitted fabric thread (3) with a right-left pattern,
d) transferring the stitches located on the first needle bed (1) onto the second needle bed (2),
e) casting off the stitches on the second needle bed (2) with the knitted fabric thread (3) with a right-left pattern,
f) knitting at least one stitch course (8, 9) with a right-right pattern on both needle beds (1, 2) with the knitted fabric thread (3),
g) introducing the elastic weft thread (4),
h) casting off the stitches on the second needle bed (2) with the knitted fabric thread (3) with a right-left pattern,
i) transferring the stitches located on the second needle bed (2) onto the first needle bed (1),
j) casting off the stitches on the first needle bed (1) with the knitted fabric thread (3) with a right-left pattern,
k) repeating steps a)-j) as many times as desired.

2. Method according to Claim 1, **characterized in that** at least two of steps a), b) and c), and/or f), g) and h) are carried out at the same time.

3. Method according to Claim 1 or 2, **characterized in that**, before the first step a) is carried out, one or more knitted ground fabric stitch courses knitted with the knitted fabric thread (3) are knitted with a knitted ground fabric pattern type, preferably with an introduced weft thread (4).

4. Method according to one of the preceding claims, **characterized in that**, after the last step j) has been carried out, one or more knitted ground fabric stitch courses knitted with the knitted fabric thread (3) are knitted with a knitted ground fabric pattern type, preferably with an introduced weft thread (4).

5. Method according to one of the preceding claims, **characterized in that** the stitch courses knitted in steps a)-j) are knitted across the entire width of the knitted article (10).

6. Method according to one of Claims 1 to 4, **characterized in that** the stitch courses knitted in steps a)-j) are knitted only partially across the width of the knitted article (10), wherein, as seen in the direction of the knitted fabric width, knitted ground fabric stitch courses with the knitted ground fabric pattern type adjoin on one or both sides of the stitch courses knitted in steps a)-j).

7. Method according to one of the preceding claims, **characterized in that**, to form one or more gussets (14), stitch courses knitted in steps a)-j) are knitted, as seen in the direction of the knitted fabric width, only partially with a varying course length, wherein a stitch course that is continuous across the entire width is knitted next to each or to a plurality of shorter stitch courses.

8. Method according to one of the preceding claims, **characterized in that** knitting is carried out with knitted fabric threads (3) having two or more different colours, thread qualities and/or thread materials.

9. Method according to one of the preceding claims, **characterized in that** a machine having a stitch transfer device is used as knitting machine.

10. Knitted article consisting of at least one knitted fabric thread (3) and at least one elastic weft thread (4), comprising at least one structural portion (7) knitted from the knitted fabric thread (3), **characterized in that** the structural portion (7) consists of a plurality of mutually parallel stitch courses which extend in a meandering manner as seen in the knitted fabric longitudinal direction (L) and stand proud towards the top side and the underside of the structural portion (7) and into which the weft thread (4) has been introduced, and which are able to be pulled apart under the action of a tensile load acting in the longitudinal direction (L) of the knitted article (10), with an elastic restoring force being built up, and which are able to be pushed together under the action of a compressive load acting in the opposite direction.

11. Knitted article according to Claim 10, **characterized in that** the structural portion (7), as seen in the knitted fabric longitudinal direction (L), adjoins at least one upstream or downstream knitted ground fabric portion (12, 13).

12. Knitted article according to Claim 10 or 11, **characterized in that** the structural portion (7) extends across the entire width (Q) of the knitted article (10) or only partially across the width (Q) of the knitted article (10).

13. Knitted article according to one of Claims 10 to 12, **characterized in that** at least one gusset (14) has been knitted in the structural portion (7).

14. Knitted article according to one of Claims 10 to 13, **characterized in that** it is knitted from knitted fabric threads (3) having at least two different colours, thread qualities and/or thread materials.

## Revendications

1. Procédé de fabrication d'une pièce tricotée (10) à partir d'au moins un fil à tricoter (3) formant des mailles et d'au moins un fil de trame élastique (4) sur une machine à tricoter comprenant une première et une deuxième fonture (1, 2), comprenant les étapes suivantes :
a) le tricotage d'au moins une rangée de mailles (8, 9) avec une armure endroit-endroit sur les deux fontures (1, 2) avec le fil à tricoter (3),
b) l'insertion du fil de trame élastique (4),
c) l'arrêt des mailles sur la première fonture (1) avec le fil à tricoter (3) avec une armure endroitenvers,
d) le transfert des mailles se trouvant sur la première fonture (1) sur la deuxième fonture (2),
e) l'arrêt des mailles sur la deuxième fonture (2) avec le fil à tricoter (3) avec une armure endroitenvers,
f) le tricotage d'au moins une rangée de mailles (8, 9) avec une armure endroit-endroit sur les deux fontures (1, 2) avec le fil à tricoter (3),
g) l'insertion du fil de trame élastique (4),
h) l'arrêt des mailles sur la deuxième fonture (2) avec le fil à tricoter (3) avec une armure endroitenvers,
i) le transfert des mailles se trouvant sur la deuxième fonture (2) sur la première fonture (1),
j) l'arrêt des mailles sur la première fonture (1) avec le fil à tricoter (3) avec une armure endroitenvers,
k) la répétition un nombre quelconque de fois des étapes a) à j).

2. Procédé selon la revendication 1, **caractérisé en ce qu'**au moins deux des étapes a), b) et c), et/ou f), g) et h) sont réalisées simultanément.

3. Procédé selon la revendication 1 ou 2, **caractérisé en ce qu'**avant la réalisation de la première étape a), une ou plusieurs rangées de mailles de tricot de base tricotées avec le fil à tricoter (3) sont tricotées selon un type d'armure de tricot de base, de préférence avec un fil de trame (4) inséré.

4. Procédé selon l'une quelconque des revendications précédentes, **caractérisé en ce qu'**après la réalisation de la dernière étape j), une ou plusieurs rangées de mailles de tricot de base tricotées avec le fil à tricoter (3) sont tricotées selon un type d'armure de tricot de base, de préférence avec un fil de trame (4) inséré.

5. Procédé selon l'une quelconque des revendications précédentes, **caractérisé en ce que** les rangées de mailles tricotées dans les étapes a) à j) sont tricotées sur la largeur entière de la pièce tricotée (10) .

6. Procédé selon l'une quelconque des revendications 1 à 4, **caractérisé en ce que** les rangées de mailles tricotées dans les étapes a) à j) ne sont tricotées qu'en sections sur la largeur de la pièce tricotée (10), l'un ou les deux côtés des rangées de mailles tricotées dans les étapes a) à j), tel que vu dans la direction de la largeur du tricot, étant suivis par des rangées de mailles de tricot de base présentant le type d'armure de tricot de base.

7. Procédé selon l'une quelconque des revendications précédentes, **caractérisé en ce que**, pour la formation d'un ou de plusieurs goussets (14), des rangées de mailles tricotées dans les étapes a) à j) ne sont tricotées qu'en sections avec une longueur de rangée variable, tel que vu dans la direction de la largeur du tricot, une rangée de mailles continue sur la largeur entière étant tricotée à côté de chaque ou de plusieurs rangées de mailles plus courtes.

8. Procédé selon l'une quelconque des revendications précédentes, **caractérisé en ce que** le tricotage est effectué avec deux fils de tricot (3) ou plus présentant des couleurs, des qualités de fil et/ou des matériaux de fil différents.

9. Procédé selon l'une quelconque des revendications précédentes, **caractérisé en ce qu'**une machine munie d'un dispositif de transfert de mailles est utilisée en tant que machine à tricoter.

10. Pièce tricotée constituée d'au moins un fil à tricoter (3) et d'au moins un fil de trame élastique (4), comprenant au moins une section structurée (7) tricotée à partir du fil à tricoter (3), **caractérisée en ce que** la section structurée (7) est constituée par plusieurs rangées de mailles en relief vers le côté supérieur et vers le côté inférieur de la section structurée (7), situées parallèlement les unes aux autres, s'étendant en méandres tel que vu dans la direction longitudinale du tricot (L), dans lesquelles le fil de trame (4) est inséré, et qui, sous l'action d'une sollicitation de traction agissant dans la direction longitudinale (L) de la pièce tricotée (10), peuvent être écartées les unes des autres en accumulant une force de rappel élastique, et qui, sous l'action d'une sollicitation de pression agissant en sens inverse, peuvent être déplacées les unes vers les autres.

11. Pièce tricotée selon la revendication 10, **caractérisée en ce que** la section structurée (7) est suivie, tel que vu dans la direction longitudinale du tricot (L), par au moins une section de tricot de base située en amont ou en aval (12, 13).

12. Pièce tricotée selon la revendication 10 ou 11, **caractérisée en ce que** la section structurée (7) s'étend sur la largeur entière (Q) de la pièce tricotée (10) ou uniquement en sections sur la largeur (Q) de la pièce tricotée (10).

13. Pièce tricotée selon l'une quelconque des revendications 10 à 12, **caractérisée en ce qu'**au moins un gousset (14) est tricoté dans la section structurée (7) .

14. Pièce tricotée selon l'une quelconque des revendications 10 à 13, **caractérisée en ce qu'**elle est tricotée à partir d'au moins deux fils de tricot (3) présentant des couleurs, des qualités de fil et/ou des matériaux de fil différents.
